# EUROPÄISCHE PATENTSCHRIFT

(11) **EP 0 711 294 B1**
(45) Veröffentlichungstag und Bekanntmachung des Hinweises auf die Patenterteilung: **14.02.2001**
(21) Anmeldenummer: 95920883.6
(22) Anmeldetag: 25.05.1995
(51) Int. Cl.: C07D 487/04

(54) **DIAZAPENTALENDERIVATE ALS SPEZIFISCHES REAGENZ FÜR THIOLVERBINDUNGEN**
USE OF DIAZAPENTALENE DERIVATIVES AS REAGENTS SPECIFIC FOR THIOL COMPOUNDS
DERIVES DE DIAZAPENTALENE UTILISES COMME REACTIFS SPECIFIQUES DES COMPOSES DE THIOL

(30) Priorität: 01.06.1994 DE 4419097
(43) Veröffentlichungstag der Anmeldung: 15.05.1996
(73) Patentinhaber: Roche Diagnostics GmbH, 68298 Mannheim (DE)
(72) Erfinder: GOMPPER, Rudolf, D-81247 München (DE); KUBBIES, Manfred, D-82377 Penzberg (DE); SCHMIDT, Axel, D-80634 München (DE); VIRNEKAS, Bernhard, D-80333 München (DE)
(86) Internationale Anmeldenummer: EP9501981
(87) Internationale Veröffentlichungsnummer: WO9532969

(56) Entgegenhaltungen:
- ANGEWANDTE CHEMIE INTERNATIONAL EDITION., Bd. 27, Nr. 8, 1988 WEINHEIM DE, Seiten 842-845, F. CLOSS ET AL. '1,3,4,6-Tetraamino-2,5-diazapentalenes : Delocalized antiaromatic compounds'

## Beschreibung

Die Erfindung betrifft Diazapentalenderivate und deren Verwendung als thiolgruppenspezifisches Fluorochrom, insbesondere zur Zellanfärbung, sowie ein Verfahren zum fluorimetrischen Nachweis von thiolgruppenenthaltenden Verbindungen, insbesondere in Zellen.

Von zahlreichen bekannten fluoreszierenden Verbindungen, die mit Thiolgruppen reagieren, erfüllen nur wenige die Bedingung, daß diese Reaktion in genügend selektiver Weise erfolgt.

Ein wichtiges Anwendungsgebiet selektiv mit Thiolgruppen reagierender Fluorochrome ist allgemein die Analytik von Verbindungen, die Thiolgruppen enthalten (insbesondere Proteine), zum Beispiel die Detektion solcher Verbindungen in der HPLC-Analytik, in der Dünnschichtchromatographie oder in der Gelelektrophorese.

Ein spezielles Anwendungsgebiet solcher thiolgruppenspezifischer Reagenzien ist die Anfärbung von lebenden Zellen. Dabei müssen die fluorochromen thiolgruppenspezifischen Reagenzien in die Zelle eindringen können, um mit den dort vorhandenen Thiolen, insbesondere Glutathion und Cysteinresten, reagieren zu können. Durch Messung der Fluoreszenz der so angefärbten Zellen kann der Gehalt oder unter Umständen auch die Position von Thiolen, insbesondere Glutathion, in der Zelle bestimmt werden. Da Glutathion in der Zelle u.a. die Funktion hat, Mutagene oder Karzinogene unschädlich zu machen, liefert die Bestimmung des zellulären Gehalts von Glutathion auch eine Aussage über den Schutz einer Zelle gegenüber schädlichen Einflüssen. Ferner sind zelluläre Thiole, insbesondere Glutathion, in einer Reihe weiterer biochemischer Vorgänge involviert, so daß ihr zellulärer Gehalt mit einer Reihe pathologischer Krankheiten in Verbindung gebracht werden kann (siehe beispielsweise J. Bio. Chem. 263, 17205).

Nach Inkubation der Zellen mit einem thiolspezifischen Fluorochrom erfolgt die Bestimmung des Gehalts an fluoreszierenden Thioladdukten in der Zelle bevorzugt mittels "flow cytometry" oder auch "digitalem Videoimaging". Flow Cytometry hat die sehr vorteilhafte Eigenschaft, physiologische Parameter in einer großen Anzahl lebender Einzelzellen messen zu können. Sie erlaubt sogar Heterogenitäten innerhalb einer Zellpopulation zu bestimmen. Bei diesem Verfahren wird die Fluoreszenz einzelner Zellen in einem Flüssigkeitsstrom bevorzugt nach Anregung mit einem Argonlaser bei 488 nm mittels einer Fluoreszenzoptik bestimmt (Flow Cytometry and Sorting, M.R. Melamed et al. (eds) Wiley and Liss, 1990). Eine Übersicht zur Bestimmung von Thiolen in Zellen durch flow cytometry geben Rabinowitch et al. in Clinical Flow Cytometry, K.D. Bauer et al. (eds) Williams and Wilkins, 1993, Seite 505 - 534, Durant et al., Radiation Research 95, 456-470 (1983) und McLean Grogan, Guide to Flow Cytometry Methods, Marcel Dekker Inc., New York and Basel, (1990), Seite 171-176. Diese Literaturstellen geben auch eine Übersicht über gebräuchliche fluorochrome thiolgruppenspezifische Reagenzien für die Flow-Cytometrie. Am gebräuchlichsten werden darin Maleimidderivate und Brombimane beschrieben.

Nachteile der im Stand der Technik beschriebenen fluorochromen thiolspezifischen Reagenzien zur Zellfärbung sind, daß sie zum Teil eine hohe Zelltoxizität aufweisen (Brombimane), nur schwer in die Zellen eindringen (Fluorescein-5-maleimid, Didansylcystin), eine für die Anregungswellenlänge insbesondere von gebräuchlichen Argonlasern ungünstige Anregungswellenlänge haben (Bimane bei 360-400 nm, 3-4-Maleimidylphenyl-4-methyl-7-diethylamino-coumarin (CPM): bei 395 nm, Didansylcystin: 335 nm), eine langsame Additionskinetik besitzen (Chlorbiman, CPM) und deswegen in hohen Konzentrationen eingesetzt werden müssen, oder auch eine ungenügende Spezifität für Thiolgruppen besitzen (Orthophthaldialdehyd). Ferner zeigen die meisten bekannten Verbindungen eine geringe Photostabilität, was insbesondere bei mikroskopischer Betrachtung der Zellen z.B. unter einer Quecksilberdampflampe über einen längeren Zeitraum einen Nachteil darstellt.

Die Aufgabe der vorliegenden Erfindung bestand daher darin, die Nachteile der thiolspezifischen fluorochromen Verbindungen des Standes der Technik zu beseitigen und fluorochrome Reagenzien zur Verfügung zu stellen, die sehr spezifisch und schnell mit Thiolgruppen reagieren, die schnell in Zellen eindringen und geringe Zelltoxität aufweisen, die bei einer für einen Argonlaser günstigen Anregungswellenlänge benutzt werden können, die eine hohe Photostabilität aufweisen und damit für die Zelleinfärbung und besonders in der Flow-Cytometrie in einer vorteilhaften Weise eingesetzt werden können.

Die Aufgabe wird gelöst durch die Erfindung, wie sie in den Ansprüchen charakterisiert ist.

Gegenstand der Erfindung sind Diazapentalenderivate der Formel I 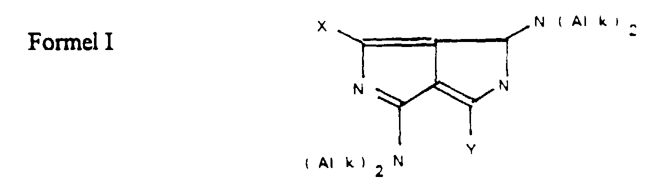 in der X und Y gleich oder verschieden sind und eine Abgangsgruppe darstellen, die weniger nucleophil ist als eine Thiolverbindung.

Solche Abgangsgruppen sind dem Fachmann bekannt. Bevorzugte Beispiele sind stickstoffhaltige Heterozyklen wie Pyrazole, Benzpyrazole, Imidazole, Benzimidazole, Triazole und Benzotriazole, Tetrazole, Pyrrol-, Indol-, Carbazol- und Pyrimidingruppen, aromatische Abgangsgruppen wie Hydroxy-, Alkoxy-, Carboxy-, Alkoxycarbonyl-, Nitro-, Amino-, Alkylamino-, Dialkylamino-substituiertes Phenyl oder Pyridin, Triphenylphosphonium, Tosyl und Halogenide und Pseudohalogenide wie Fluor, Chlor, Brom, Jod, SCN, N₃ usw.

Ganz besonders bevorzugt als Abgangsgruppe ist die Imidazolgruppe.

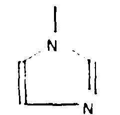

Die Alkylgruppen am Aminostickstoff haben 1-6 C-Atome, bevorzugt ist die Methylgruppe.

Die aromatischen oder heteroaromatischen Abgangsgruppen können selbstverständlich noch substituiert sein, insbesondere durch solche Gruppen, die die Abgangsgruppen weniger nucleophil machen. Bei stickstoffhaltigen Heteroaromaten können beispielsweise Stickstoffatome substituiert, insbesondere alkyliert sein. Solange diese Substituenten die Abgangsgruppe nicht nucleophiler machen als eine Thiolgruppe, ist die Art der Substituenten für die Erfindung nicht wesentlich.

Unter Alkylgruppe wird bevorzugt eine C1-C6 Alkylgruppe verstanden.

Ein weiterer Gegenstand der Erfindung ist ein Verfahren zum spezifischen Nachweis von thiolgruppenenthaltenden Verbindungen in einer Probe durch Umsetzung der Probe mit einem mit Thiolgruppen reagierenden Fluorochrom und Messung der Fluoreszenz der Reaktionsprodukte als Maß für die Anwesenheit oder Menge der Thiolverbindung in der Probe, dadurch gekennzeichnet, daß das mit einer Thiolgruppe reagierende Fluorochrom ein Diazapentalenderivat gemäß Formel I ist.

Das Verfahren wird so durchgeführt, daß die erfindungsgemäßen Fluorochrome bevorzugt in einem Überschuß mit nachzuweisenden Thiolverbindungen in Lösung, bevorzugt in wässriger Lösung, inkubiert werden und die gebildeten fluoreszierenden Produkte gemessen werden. Die Anregungswellenlänge liegt bei der Messung dabei bevorzugt zwischen 460 und 500 nm, die Meßwellenlänge bevorzugt bei über 510 nm.

Die nachzuweisenden Verbindungen können freie Thiolgruppen an sich schon enthalten oder es können durch bekannte Methoden Thiolgruppen in die Verbindungen chemisch eingeführt werden, um sie dann mit den erfindungsgemäßen Fluorochromen nachweisen zu können.

Damit eignen sich die erfindungsgemäßen Fluorochrome auch sehr gut als fluoreszierende Markermoleküle für thiolgruppenenthaltende Moleküle oder für Moleküle, die mit Thiolgruppen modifiziert wurden, (z.B. thiolmodifizierte Nukleotide, Proteine, thiolmodifizierte Lipide) und insbesondere als fluoreszierende Konjugate von Haptenen, Antigenen oder Antikörpern für Immunoassays.

Vorteilhaft kann das erfindungsgemäße Nachweisverfahren von Verbindungen, die Thiolgruppen enthalten, zum qualitativen aber auch quantitativen Nachweis einer oder mehrerer thiolgruppenenthaltender Substanzen bei einer chromatographischen Trennung, z.B. einer HPLC-Trennung oder eine Gelelektrophorese angewandt werden, indem die Probe mit den erfindungsgemäßen Fluorochromen vor der Trennung umgesetzt wird und die thiolgruppenenthaltenden Verbindungen, zum Beispiel Proteine, während der chromatographischen Trennung, fluorometrisch detektiert werden. Bei einer dünnschichtchromatographischen Trennung können die aufgetrennten Thiolverbindungen dagegen bevorzugt durch Besprühen mit den erfindungsgemäßen Fluorochromen auf der Dünnschichtplatte detektierbar gemacht werden.

Ein weiterer Gegenstand der Erfindung ist ein Verfahren zur spezifischen Bestimmung von thiolgruppenenthaltenden Bestandteilen einer Zelle durch Inkubation der Zellen mit einem mit Thiolgruppen reagierenden Fluorochrom und Messen der Fluoreszenz der Reaktionsprodukte als Maß für die Anwesenheit oder Menge der Thiolverbindungen in der Zelle, dadurch gekennzeichnet, daß das mit einer Thiolgruppe reagierende Fluorochrom eine Verbindung gemäß Formel I ist.

Das Verfahren wird so durchgeführt, daß Zellen mit dem erfindungsgemäßen Fluorochrom bevorzugt in einer wässrigen Lösung inkubiert werden.

Die Zellkonzentration liegt dabei üblicherweise zwischen 10⁴ und 10⁸ Zellen pro ml, bevorzugt zwischen 10⁵ und 10⁷ Zellen pro ml.

Die Konzentration des erfindungsgemäßen Fluorochroms liegt abhängig vom Zelltyp, Zellgröße und dem zellphysiologischen Zustand zwischen 10 ng/ml und 10 ug/ml, bevorzugt zwischen 50 ng und 500 ng / ml, ganz besonders bevorzugt bei 70 - 200 ng/ml. Dies sind geringere Konzentrationen, die zur Anfärbung der Zelle nötig sind als aus dem Stand der Technik bekannt.

Die Inkubationszeit liegt im allgemeinen zwischen 5 und 30 Minuten. Schon nach 10 Minuten kann eine fast vollständige Reaktion erreicht werden.

Die Messung der Fluoreszenz kann über verschiedene Verfahren erfolgen, z. B. Fluoreszenzmikroskopie. Dabei werden die Zellen unter den oben genannten bevorzugten Bedingungen mit dem erfindungsgemäßen Fluorochrom markiert, auf einen Objektträger aufgebracht und mit einem Deckglas bedeckt. Anschließend werden die markierten Zellen mit einem konventionellen Fluoreszenzmikroskop betrachtet, wobei die Anregungswellenlänge bevorzugt zwischen 460 und 500 nm liegt. Für die Fluorochromanregung kommen in Frage sowohl Quecksilberhochdrucklampen als auch Xenonlampen. Die optischen Farbteiler und Emissionsfilter werden bevorzugt so gewählt, daß die emittierte Fluoreszenz ab einer Wellenlänge größer 510 nm für die visuelle Betrachtung oder Kameraaufnahme für die quantitative digitale Imagezytometrie zur Verfügung steht.

In einem besonderen Verfahren können die Zellen auch mittels konfokaler, hochauflösender Mikroskopie analysiert und die Fluoreszenz quantifiziert werden. Je nach konfokaler Meßtechnik kommen als Anregungslichtquellen wiederum die oben genannten Lichtquellen, oder aber Laser mit Emissionswellenlängen im oben genannten Bereich in Frage.

Besonders bevorzugt ist die Messung mittels Flow-Cytometrie, bei der einzelne Zellen mit hoher Flußgeschwindigkeit bezüglich ihrer Fluoreszenzeigenschaften analysiert werden.

Die Anregungswellenlängen bei allen Fluoreszenzmessungen liegt bevorzugt zwischen 460 und 500 nm, der Anregungswellenlänge des Argonlasers. Die Messwellenlänge liegt bevorzugt über 510 nm.

Die erfindungsgemäßen Fluorochrome färben die Zellen mit einer hohen Thiolspezifität an. Sie weisen nur eine geringe Cytotoxizität auf (> 100 ng / ml) und zeigen eine schnelle Sättigungskinetik, d.h. bei nur 100 ng / ml Fluorochrom, das mit nur 10⁶ Zellen inkubiert wird, ist die Sättigung schon nach 10 min erreicht (80 - 90% der maximalen Fluoreszenz). Zudem zeigen die fluorochrom markierten Zellen eine hohe Photostabilität.

Ein weiterer Gegenstand der Erfindung ist die Verwendung der erfindungsgemäßen thiolspezifischen Fluorochrome zur Bestimmung von thiolgruppenenthaltenden Verbindungen.

Ein weiterer Gegenstand der Erfindung ist die Verwendung der erfindungsgemäßen Fluorochrome für die Flow-Cytometrie und für die Zelldiagnostik.

Ein weiterer Gegenstand der Erfindung ist ein Verfahren zur Herstellung der erfindungsgemäßen Fluorochrome.

Die erfindungsgemäßen Fluorochrome werden durch Umsetzung von einem Äquivalent Dichlordiazapentalen mit zwei Äquivalenten eines Nucleophils X' und / oder Y', das nucleophiler als Chlor, aber weniger nucleophil als eine Thiolverbindung ist, hergestellt. Die Darstellung der Ausgangsverbindung Dichlordiazapentalen ist in F. Closs et al., Angew. Chem. 100, 875 (1988) beschrieben.

Die Reaktion der erfindungsgemäßen Fluorochrome mit Thiolgruppen erfolgt dabei so, daß hauptsächlich nur eine Abgangsgruppe X oder Y durch einen an eine Thiolgruppe gebundenen Rest R-SH substituiert wird. Man erhält dabei hauptsächlich die Thioether-Verbindungen III, die starke Fluoreszenz mit einer hohen Quantenausbeute aufweisen. Bei längerer Reaktionszeit, insbesondere bei höherer Temperatur als Raumtemperatur, können auch beide Abgangsgruppen X und Y durch einen an eine Thiolgruppe gebundenen Rest substituiert werden.

Ein weiterer Gegenstand der Erfindung sind auch die resultierenden fluoreszierenden Verbindungen der Formel III und IV, 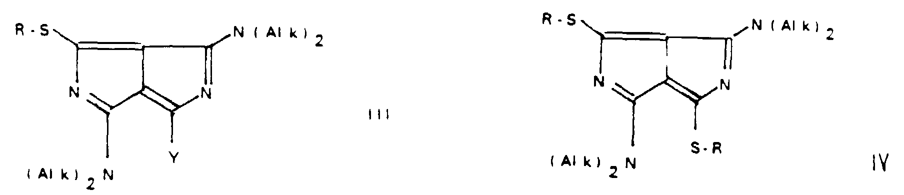 in denen Y die oben gegebenen Definition besitzt und R ein an eine Thiolgruppe gebundener Rest eines zu detektierenden Moleküls wie zum Beispiel ein Protein, ein (modifiziertes) Nukleotid, ein (modifiziertes) Lipid, Glutathion, ein Hapten, ein Antikörper oder ein Antigen ist.

Ein weiterer Gegenstand der Erfindung ist die Verwendung der erfindungsgemäßen Fluorochrome als Marker thiolgruppenenthaltender Verbindungen bzw. die Verwendung der Verbindungen gemäß Formel III oder IV als fluoreszierende Konjugate z.B. in Immunoassays.

### Beispiel 1

### Anwendungsbeispiel Flow-Cytometrie:

Jurkat T-Zellen werden zu 10⁶ Zellen pro ml in PBS resuspendiert und mit 100 ng / ml des Fluorochroms Diimidazolodiazapentalen versetzt. Die Zellen werden bei 37°C für 10 Minuten inkubiert. Da die Fluorochrome, die nicht mit SH-Gruppen reagierten, nicht fluoreszieren, ist keine Waschung, d.h. Entfernung der ungebundenen Fluorochrome notwendig. Die markierten Jurkat Zellen werden in einem Flowcytometer (z.B. Cytofluorograph, Ortho Instruments oder FACSstar plus, Becton Dickinson) analysiert, wobei die Fluorochromanregung mittels eines Argonlasers 488 nm und 50mW erfolgt und die Zellflußrate bei 600 Zellen pro Sekunde liegt. Die emittierte Fluoreszenz wird über einen Langpaßemissionsfilter gemessen, welcher einen Duchlaßbereich größer 510 nm hat. Die Intensitätsverteilung der Zellen wird mittels eines eindimensionalen Fluoreszenzhistograms dargestellt.

Zum Ausschluß der unspezifischen Markierung der Zellen wird eine lebend/tot Gegenfärbung mit Propidium Jodid durchgeführt, wobei ca. 1 ug/ ml dieses Fluorochromes schon während der Inkubation mit dem erfindungsgemäßen Fluorochrom oder aber kurz vor der Messung zugegeben wird. Die Datenanalyse erfolgt dann über ein zweidimensionales Zytogramm.

Desweiteren besteht die Möglichkeit, weitere Fluoreszenzzellmarkierungen zusammen mit dem erfindungsgemäßen Fluorochrom durchzuführen, wobei die Emissionswellenlänge der weiteren Fluorochrome bevorzugt über 580 nm liegen soll. Insbesondere kommt die Zellmarkierung mittels monoklonaler Antikörper, welche wiederum fluoreszenzmarkiert sind zur Anwendung (z.B. Phycoerythrin).

### Beispiel 2

### Synthese von 1,4-Bis(dimethylamino)-3,6-diimidazol-1-pyrrolo(3,4-c) pyrrol

4.00 g (15.4 mmol) 1,4-Bis(dimethylamino)-3,6-dichloro-1-pyrrolo(3,4-c) pyrrol wurden in 100 ml Acetopnitril mit 6.81 g (100 mmol) Imidazol versetzt und das Gemisch 2 h lang bei 60° C gerührt. Nach Abkühlung auf 0° C wurde der Niederschlag abgesaugt und mit Acetonitril und Ether gewaschen. Ausb: 4,77 g (96%) rote, grün glänzende Kristalle (aus Acetonitril), Schmp. 259° C (Zers).

IR(Nujol): v = 3100 cm⁻¹, 1599, 1540, 1408, 1331, 1312, 1279, 1050, 988, 918. -UV/Vis (CH3CN): max (1g ) = 275 nm (4.114), 304 (sh), 470 (sh), 501 (4.445).- UV/Vis (CH3COOH): max (qual.) = 218 nm, 268, 310 (sh), 476 (sh), 507. -1H-NMR (CDCI3): δ= 3.05 (s, 12 H, NCH3), 7.13 (dd, 2H, 4'-H oder 5'-H), 7.36 (dd, 2H,, 4'H oder 5'H), 7.91 (dd, 2H,2'H)-13C-NMR (CDC3): δ=-40.69 (q, NCH3), 109.99 (s,C-3a, C-6a), 120.27 (d, C-5'), 129.47 (d, C-4'), 138.09 (d, C-2'), 149.73 (s, C-3, C-6), 158.55 (s, C-1, C-4). MS (70 eV), m/z (%) [M*], 307 (8) [M*-CH3], 322 (100) [M*].

## Patentansprüche

1. Diazapentalenderivate der Formel I' als thiolgruppenspezifische Fluorochrome, in denen X' und Y', die gleich oder verschieden sein können, eine Abgangsgruppe bedeuten, die weniger nucleophil ist als eine Thiolverbindung, wobei für X' und Y' Chlor ausgenommen ist und Alk eine Alkylgruppe mit 1 bis 6 C-Atomen bedeutet.

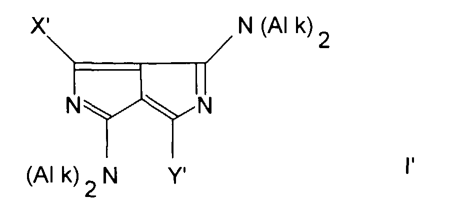

2. Diazapentalenderivate gemäß Anspruch 1, dadurch gekennzeichnet, daß die Abgangsgruppen ein Pyrazol, Benzopyrazol, Imidiazol, Benzimidazol, Triazol, Benzotriazol, Tetrazol, eine Pyrrol-, Indol-, Carbozol- oder Pyrimidingruppe, eine Hydroxy-, Alkoxy-, Alkoxycarbonyl-, Carboxy-, Amino-, Monoalkylamino-, Dialkylamino- oder Nitro-substituierte Phenylgruppe oder Pyridingruppe, Triphenylphosphonium, Tosyl, Fluor, Brom, Jod, SCN, Azid darstellt.

3. Diazapentalenderivate gemäß Anspruch 2, dadurch gekennzeichnet, daß die Abgangsgruppe einen Imidazolrest darstellt.

4. Verfahren zum spezifischen Nachweis von Verbindungen, die Thiolgruppen enthalten, in einer Probe durch Umsetzung der Probe mit einem mit Thiolgruppen reagierenden Fluorochrom und Messung der Fluoreszenz der Reaktionsprodukte als Maß für die Anwesenheit oder Menge der Thiolverbindungen in der Probe, dadurch gekennzeichnet, daß das Fluorochrom ein Diazapentalenderivat gemäß Formel I ist, in denen X und Y, die gleich oder verschieden sein können, eine Abgangsgruppe bedeuten, die weniger nucleophil ist als eine Thiolverbindung und Alk eine Alkylgruppe mit 1 bis 6 C-Atomen bedeutet.

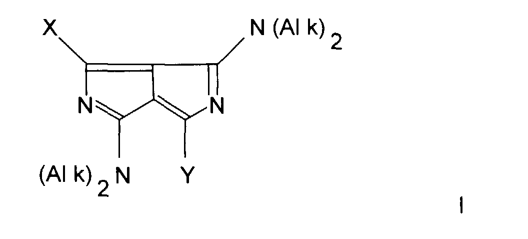

5. Verfahren gemäß Anspruch 4, dadurch gekennzeichnet, daß die Abgangsgruppe ein Pyrazol, Benzopyrazol, Imidiazol, Benzimidazol, Triazol, Benzotriazol, Tetrazol, eine Pyrrol-, Indol-, Carbazol- oder Pyrimidingruppe, eine Hydroxy- Alkoxy-, Alkoxycarbonyl-, Carboxy-, Amino-, Alkylamino, Dialkylamino oder Nitro-substituierte Phenylgruppe oder Pyridingruppe, Triphenylphosphonium, Tosyl, Fluor, Chlor, Brom, Jod, SCN, Azid darstellt.

6. Verfahren gemäß Anspruch 5, dadurch gekennzeichnet, daß die Abgangsgruppe ein Imdidazol darstellt.

7. Verfahren gemäß Anspruch 2, dadurch gekennzeichnet, daß bei einer Wellenlänge zwischen 460 und 500 nm angeregt wird und bei über 510 nm gemessen wird.

8. Verfahren gemäß Anspruch 4 bis 7, dadurch gekennzeichnet, daß die Detektion verschiedener thiolgruppenenthaltender Verbindungen während oder nach einer chromatographischen Trennung erfolgt.

9. Verfahren zur spezifischen Bestimmung von Bestandteilen einer Zelle, die Thiolgruppen enthalten, durch Inkubation der Zellen mit einem mit Thiolgruppen reagierenden Fluorochrom und Messen der Fluoreszenz der Reaktionsprodukte als Maß für die Anwesenheit oder Menge der Thiolverbindung in der Zelle, dadurch gekennzeichnet, daß das mit einer Thiolgruppe reagierende Fluorochrom eine Verbindung gemäß Formel I ist.

10. Verfahren gemäß Anspruch 9, dadurch gekennzeichnet, daß das Fluorchrom in einer Konzentration von 10 ng / ml bis 10 µg / ml eingesetzt wird.

11. Verfahren gemäß Anspruch 9 oder 10, dadurch gekennzeichnet, daß die Detektion über Fluoreszenzmikroskopie erfolgt.

12. Verfahren gemäß Anspruch 9 oder 10, dadurch gekennzeichnet, daß die Detektion mittels digitalem Videoimaging erfolgt.

13. Verfahren gemäß Anspruch 9 oder 10, dadurch gekennzeichnet, daß die Detektion mittels konfokaler Mikroskopie erfolgt.

14. Verfahren gemäß Ansprüchen 4-13, dadurch gekennzeichnet, daß Glutathion bestimmt wird.

15. Verwendung der Verbindungen gemäß Formel I zur Bestimmung von thiolgruppenenthaltenden Verbindungen.

16. Verwendung der Verbindungen gemäß Formel I für die Zellanfärbung.

17. Verwendung der Verbindungen gemäß Formel I für die Zelldiagnostik.

18. Verwendung der Verbindungen gemäß Formel I als Marker thiolgruppenenthaltender Verbindungen.

19. Verfahren zur Herstellung der Verbindungen gemäß Anspruch 1, dadurch gekennzeichnet, daß ein Äquivalent Dichlordiazapentalen der Formel II' mit zwei Äquivalenten eines Nucleophils X' und / oder Y', das nucleophiler als Chlorid aber weniger nucleophil als eine Thiolverbindung ist, umgesetzt wird.

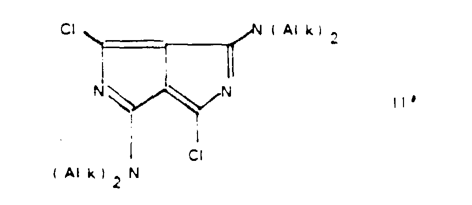

20. Diazapentalenthioether der Formel III und IV, in denen Y die Bedeutung gemäß Anspruch 4 besitzt und R ein über Fluoreszenz nachzuweisender Analyt ist.

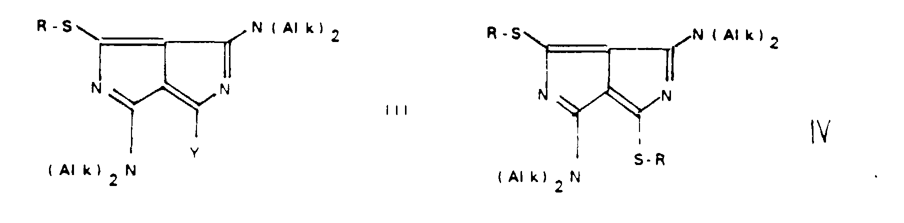

21. Diazapentalenthioether gemäß Anspruch 20, in denen R ein Protein, ein Nucleotid, ein Hapten, ein Antikörper oder Glutathion ist.

## Claims

1. Diazapentalene derivatives of formula I' as thiol group-specific fluorochromes in which X' and Y', which can be the same or different, denote a leaving group that is less nucleophilic than a thiol compound wherein X' and Y' do not denote chlorine and Alk denotes an alkyl group with 1 to 6 C atoms.

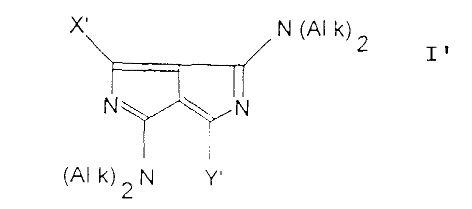

2. Diazapentalene derivatives as claimed in claim 1, wherein the leaving groups represent a pyrazole, benzopyrazole, imidazole, benzimidazole, triazole, benzotriazole, tetrazole, a pyrrole, indole, carbozole or pyrimidine group, a phenyl group or pyridine group substituted with hydroxy, alkoxy, alkoxycarbonyl, carboxy, amino, monoalkylamino, dialkylamino or nitro, triphenylphosphonium, tosyl, fluorine, bromine, iodine, SCN, azide.

3. Diazapentalene derivatives as claimed in claim 2, wherein the leaving group represents an imidazole residue.

4. Method for the specific detection of compounds containing thiol groups in a sample by reacting the sample with a fluorochrome that reacts with thiol groups and measuring the fluorescence of the reaction products as a measure for the presence or the amount of thiol compounds in the sample, wherein the fluorochrome is a diazapentalene derivative according to formula I in which X and Y, which can be the same or different, denote a leaving group that is less nucleophilic than a thiol compound and Alk denotes an alkyl group with 1 to 6 C atoms.

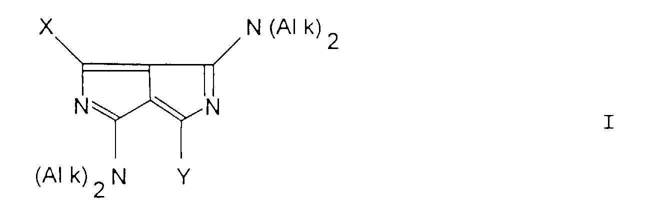

5. Method as claimed in claim 4, wherein the leaving group represents a pyrazole, benzopyrazole, imidazole, benzimidazole, triazole, benzotriazole, tetrazole, a pyrrole, indole, carbazole or pyrimidine group, a phenyl or pyridine group substituted with hydroxy, alkoxy, alkoxycarbonyl, carboxy, amino, alkylamino, dialkylamino or nitro, triphenylphosphonium, tosyl, fluorine, chlorine, bromine, iodine, SCN, azide.

6. Method as claimed in claim 5, wherein the leaving group represents an imidazole.

7. Method as claimed in claim 2, wherein it is excited at a wavelength between 460 and 500 nm and it is measured above 510 nm.

8. Method as claimed in claims 4 to 7, wherein various compounds containing thiol groups are detected during or after a chromatographic separation.

9. Method for the specific determination of components of a cell containing thiol groups by incubating the cells with a fluorochrome reacting with thiol groups and measuring the fluorescence of the reaction products as a measure for the presence or the amount of the thiol compound in the cell, wherein the fluorochrome reacting with a thiol group is a compound according to formula I.

10. Method as claimed in claim 9, wherein the fluorochrome is used at a concentration of 10 ng/ml to 10 µg/ml.

11. Method as claimed in claim 9 or 10, wherein it is detected by means of fluorescence microscopy.

12. Method as claimed in claim 9 or 10, wherein it is detected by means of digital video imaging.

13. Method as claimed in claim 9 or 10, wherein it is detected by means of confocal microscopy.

14. Method as claimed in claims 4-13, wherein glutathione is determined.

15. Use of compounds according to formula I for the determination of compounds containing thiol groups.

16. Use of the compounds according to formula I for staining cells.

17. Use of the compounds according to formula I for cell diagnostics.

18. Use of the compounds according to formula I as markers of compounds containing thiol groups.

19. Process for the production of compounds as claimed in claim 1, wherein one equivalent of dichlorodiazapentalene of formula II' is reacted with two equivalents of a nucleophile X' and/or Y' which is more nucleophilic than chloride but less nucleophilic than a thiol compound.

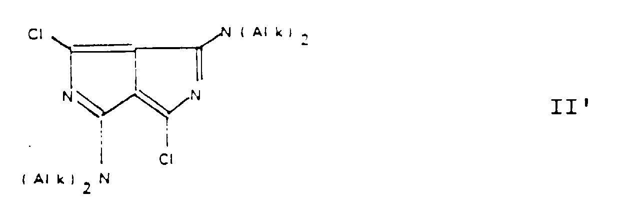

20. Diazapentalene thioether of formula III and IV in which Y has the meaning as claimed in claim 4 and R is an analyte to be detected by means of fluorescence.

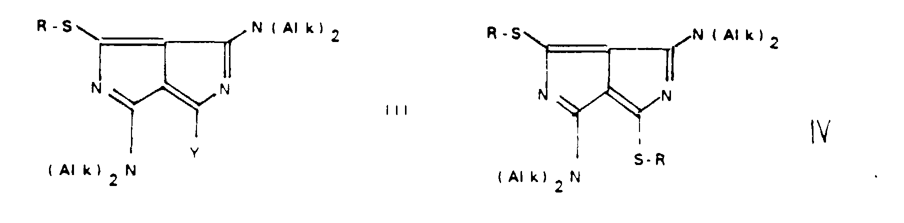

21. Diazapentalene thioether as claimed in claim 20 in which R is a protein, a nucleotide, a hapten, an antibody or glutathione.

## Revendications

1. Dérivés de diazapentalène de formule I' comme fluorochromes spécifiques des groupes thiol, dans laquelle X' et Y', qui peuvent être identiques ou différents, représentent un groupe qui se sépare, qui est moins nucléophile qu'un composé thiol, le chlore étant exclu pour X' et Y' et Alk étant un groupe alkyle comprenant 1 à 6 atomes de carbone.

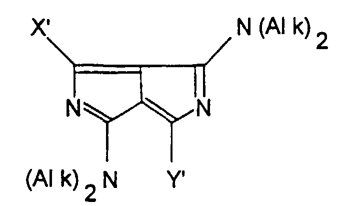

2. Dérivés de diazapentalène selon la revendication 1, caractérisés en ce que les groupes qui se séparent représentent un groupe pyrazole, un groupe benzopyrazole, un groupe imidazole, un groupe benzimidazole, un groupe triazole, un groupe benzotriazole, un groupe tétrazole, un groupe pyrrole, un groupe indole, un groupe carbazole, un groupe pyrimidine, un groupe phényle ou un groupe pyridine substitué par un groupe hydroxy, un groupe alkoxy, un groupe alkoxycarbonyle, un groupe carboxy, un groupe amino, un groupe monoalkylamino, un groupe dialkylamino ou un groupe nitro, un groupe triphénylphosphonium, un groupe tosyle, un atome de fluor, un atome de brome, un atome d'iode, un groupe SCN, un groupe azide.

3. Dérivés de diazapentalène selon la revendication 2, caractérisés en ce que le groupe qui se sépare représente un résidu imidazole.

4. Procédé pour la mise en évidence spécifique de composés qui contiennent des groupes thiol, dans un échantillon par transformation de l'échantillon avec un fluorochrome réagissant avec les groupes thiol et mesure de la fluorescence des produits de réaction comme indication de la présence ou de la quantité de composés thiol dans l'échantillon, caractérisé en ce que le fluorochrome est un dérivé de diazapentalène selon la formule I, dans lequel X et Y, qui peuvent être identiques ou différents, signifient un groupe qui se sépare qui est moins nucléophile qu'un composé thiol et Alk signifie un groupe alkyle comprenant 1 à 6 atomes de carbone.

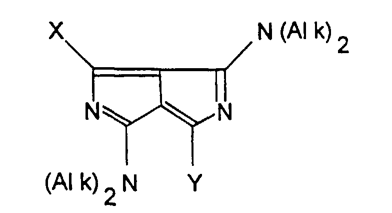

5. Procédé selon la revendication 4, caractérisé en ce que le groupe qui se sépare représente un groupe pyrazole, un groupe benzopyrazole, un groupe imidazole, un groupe benzimidazole, un groupe triazole, un groupe benzotriazole, un groupe tétrazole, un groupe pyrrole, un groupe indole, un groupe carbazole, un groupe pyrimidine, un groupe phényle ou un groupe pyridine substitué par un groupe hydroxy, un groupe alkoxy, un groupe alkoxycarbonyle, un groupe carboxy, un groupe amino, un groupe alkylamino, un groupe dialkylamino ou un groupe nitro, un groupe triphénylphosphonium, un groupe tosyle, un atome de fluor, un atome de chlore, un atome de brome, un atome d'iode, un groupe SCN, un groupe azide.

6. Procédé selon la revendication 5, caractérisé en ce que le groupe qui se sépare est un groupe imidazole.

7. Procédé selon la revendication 2, caractérisé en ce qu'on effectue l'excitation à une longueur d'onde entre 460 et 500 nm et la mesure à une longueur d'onde supérieure à 510 nm.

8. Procédé selon la revendication 4 à 7, caractérisé en ce que la détection des différents composés contenant des groupes thiol est effectuée pendant ou après une séparation chromatographique.

9. Procédé pour la détermination spécifique de constituants d'une cellule, qui contiennent des groupes thiol, par incubation des cellules avec un fluorochrome réagissant avec des groupes thiol et mesure de la fluorescence des produits de réaction comme indication de la présence ou de la quantité de composé thiol dans la cellule, caractérisé en ce que le fluorochrome réagissant avec des groupes thiol est un composé selon la formule I.

10. Procédé selon la revendication 9, caractérisé en ce que le fluorochrome est utilisé en une concentration de 10 ng/ml à 10 µg/ml.

11. Procédé selon la revendication 9 ou 10, caractérisé en ce que la détection est effectuée par microscopie de fluorescence.

12. Procédé selon la revendication 9 ou 10, caractérisé en ce que la détection est effectuée par des images vidéo digitalisées.

13. Procédé selon la revendication 9 ou 10, caractérisé en ce que la détection est effectuée par microscopie confocale.

14. Procédé selon les revendications 4 à 13, caractérisé en ce qu'on détermine le glutathion.

15. Utilisation des composés selon la formule I pour la détermination de composés contenant des groupes thiol.

16. Utilisation des composés selon la formule I pour la coloration de cellules.

17. Utilisation des composés selon la formule I pour le diagnostic cellulaire.

18. Utilisation des composés selon la formule I comme marqueur de composés contenant des groupes thiol.

19. Procédé pour la préparation de composés selon la revendication 1, caractérisé en ce qu'on fait réagir un équivalent de dichlorodiazapentalène de formule II' avec deux équivalents d'un nucléophile X' et/ou Y', qui est plus nucléophile que le chlore mais moins nucléophile qu'un composé thiol.

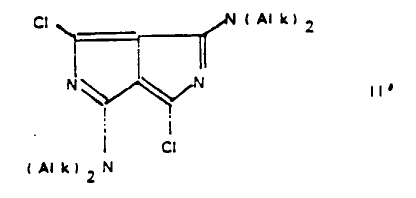

20. Diazapentalènethioéther de formule III et IV, dans lesquelles Y a la signification selon la revendication 4 et R est un analyte pouvant être mis en évidence par fluorescence.

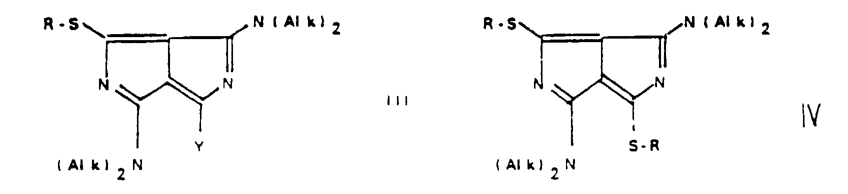

21. Diazapentalènethioéther selon la revendication 20, dans lequel R est une protéine, un nucléotide, un haptène, un anticorps ou du glutathion.
